# EUROPÄISCHE PATENTSCHRIFT

(11) **EP 2 371 939 B1**
(45) Veröffentlichungstag und Bekanntmachung des Hinweises auf die Patenterteilung: **06.03.2013**
(21) Anmeldenummer: 10157262.6
(22) Anmeldetag: 23.03.2010
(51) Int. Cl.: C12M 1/00

(54) **Rohrreaktorsystem mit einer Vielzahl von parallel zueinander angeordneten Rohrleitungen**
Tube reactor system with a number of parallel tubes
Système de réacteur tubulaire doté d'un grand nombre de conduites agencées parallèlement les unes aux autres

(43) Veröffentlichungstag der Anmeldung: 05.10.2011
(73) Patentinhaber: Georg Fischer DEKA GmbH, 35232 Dautphetal-Mornshausen (DE)
(72) Erfinder: Schüssler, Stephan, 35094 Caldern (DE); Brüggemann-Mortier, Klaus, 8207 Schaffhausen (CH)
(74) Vertreter: De Colle, Piergiacomo

(56) Entgegenhaltungen:
- WO-A2-2009/051478
- ES-A1- 2 150 389
- FR-A1- 2 685 344

## Beschreibung

Die Erfindung bezieht sich auf ein Rohrreaktorsystem umfassend eine Vielzahl von parallel zueinander angeordneten Rohrleitungen, insbesondere ein Photobioreaktor, wobei die Rohrleitungen an den Rohrenden mittels Rohrbögen zur Bildung von Rohrschleifen miteinander verbindbar angeordnet sind.

Ein Photobioreaktor ist ein Reaktionsgefäss zur Durchführung von photobiologischen Reaktionen ähnlich der Photosynthese im Pflanzenreich. In einem Photobioreaktor zur Herstellung von Biotreibstoff wird beispielsweise eine Suspension oder eine wässrige Lösung von Biomasse, beispielsweise eine Algensuspension, eingesetzt um in eine vom Sonnenlicht gesteuerte Reaktion alternative Treibstoffe zu produzieren. Photobioreaktoren werden auch eingesetzt zur Herstellung von anderen Wertstoffen, wie beispielsweise Algenkonzentrat oder Nahrungsmittelzusatzstoffen. Eine Uebersicht der Forschungsergebnissen aus dem Themenkreis Photobioreaktor, Biotreibstoff, Biodiesel und Mikroalgen und ist zu finden in einem Artikel von Yusuf Chisti in "Elsevier, Biotechnology Advances 25 (2007) 294-306".

Bekannte Photobiorektoren werden als Rohrreaktoren mit einer Vielzahl von parallel zu einander angeordneten Rohrleitungen ausgeführt. Die Rohrleitungen werden an den jeweiligen Enden mittels Rohrbögen zur Bildung eines Mäanders oder einer sehr langen Rohrschleife mit einander verbunden. Der Abstand zwischen den einzelnen Rohrleitungen bestimmt den Krümmungsradius der Rohrbögen. Als Krümmungsradius wird der Radius in der Mitte der bogenförmigen Rohrleitung definiert. Der kleinstmögliche Krümmungsradius entspricht somit einem halben Rohrleitungsdurchmesser und wird erreicht, wenn zwei unmittelbar neben einander liegenden Rohrleitungen mit einem Rohrbogen verbunden werden. Je grösser der Rohrleitungsabstand, desto grösser der Krümmungsradius des Rohrbogens und desto kleiner der Strömungswiderstand in den Rohrbogen.

Gleichzeitig wird aber die benötigte Bruttofläche für den Rohrreaktor grösser und die Energieausbeute pro Aufstellungsfläche kleiner. Um eine möglichst hohe Effizienz der Energieproduktion zu erreichen wird eine möglichst dichte Anordnung der Rohrleitungen bei einem möglichst kleinem Strömungswiderstand gewünscht.

Ausgehend von diesem Stand der Technik ist es Aufgabe der Erfindung, ein Rohrreaktorsystem mit einer Vielzahl von parallel zueinander angeordneten Rohrleitungen anzugeben, das beispielsweise in der Anwendung als Photobioreaktor auf einer möglichst optimal genutzten Bruttofläche eine möglichst effiziente Energieproduktion erreicht.

Diese Aufgabe wird gelöst durch ein Rohrreaktorsystem umfassend eine Vielzahl von parallel zueinander angeordneten Rohrleitungen, insbesondere ein Photobioreaktor, wobei die Rohrleitungen an den Rohrenden mittels Rohrbögen zur Bildung von Rohrschleifen miteinander verbindbar angeordnet sind, wobei die Rohrleitungen unmittelbar nebeneinander angeordnet sind und wobei die Rohrbögen zur Bildung von Rohrschleifen mit voneinander entfernt angeordneten Rohrenden verbindbar ausgebildet sind.

Bevorzugte Weiterbildungen der Erfindung ergeben sich aus den abhängigen Ansprüchen.

Es ist von Vorteil, dass im Photobioreaktor die Verluste durch Strömungswiderstand möglichst gering gehalten werden. Dies wird dadurch erreicht, dass die Rohrbögen einen Krümmungsradius aufweisen, der anderthalb bis achteinhalb Rohrleitungsdurchmesser entspricht, wobei der Krümmungsradius in der Mitte der bogenförmigen Rohrleitung zwischen Rohrbogeninnendurchmesser und Rohrbogenaussendurchmesser gemessen wird. Dies wird auch dadurch erreicht, dass die Rohrbögen in mehreren Ebenen mit unterschiedlichen positiven oder negativen Anstellwinkeln in Bezug auf die Ebene der Rohrleitungen angeordnet sind.

Es ist auch von Vorteil, dass das Rohrreaktorsystem möglichst einfach hergestellt werden kann. Dies wird dadurch erreicht, dass die Rohrbögen in Gruppen mit einem Versatz in der Richtung der Rohrleitungsachsen angeordnet sind.

Es ist weiter auch von Vorteil, dass das Rohrreaktorsystem möglichst optimal an die Prozessbedingungen angepasst ist. Dies wird dadurch erreicht, dass die Rohrbögen aus lichtdurchlässigem PVC-U ausgebildet sind, wobei der PVC-U-Werkstoff für die Prozessbedingungen im Photobioreaktor optimalen Transmissionseigenschaften aufweist. Dies wird auch dadurch erreicht, dass die Rohrbögen einen strömungsoptimierten Verlauf des Krümmungsradius aufweisen.

Ein Ausführungsbeispiel der Erfindung wird anhand der Figuren beschrieben. Es zeigen:
Figur 1 eine ausschnittsweise perspektivische Sicht auf ein erfindungsgemässes Rohrreaktorsystem,
Figur 2 eine ausschnittsweise perspektivische Sicht auf ein weiteres Rohrreaktorsystem und
Figur 3 eine ausschnittsweise perspektivische Sicht auf ein weiteres erfindungsgemässes Rohrreaktorsystem.

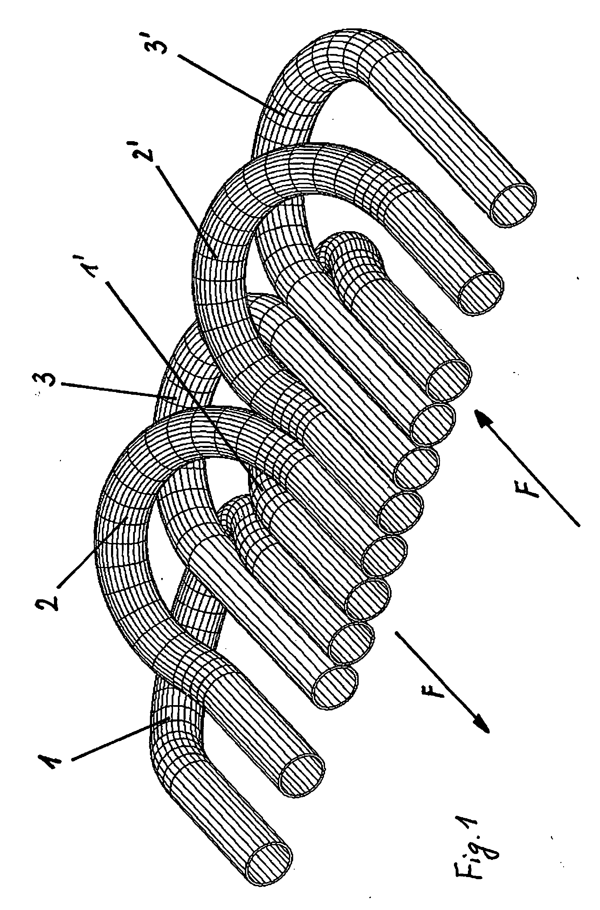

In Figur 1 ist ein Ausschnitt aus einem Rohrreaktorsystem perspektivisch dargestellt. Figur 1 zeigt sechs Rohrbögen 1, 1', 2, 2', 3 und 3', die mit den Rohrenden einer Vielzahl unmittelbar nebeneinander liegend angeordneten Rohrleitungen verbunden werden können. Die Rohrleitungen selbst, die, im Falle eines Photobioreaktors, eine beachtliche Länge von mehreren Metern aufweisen können, wurden zugunsten der Deutlichkeit aus Figur 1 weg gelassen. In Figur 1 wird mit einem Pfeil F die Strömungsrichtung in den Rohrleitungen angedeutet.

Aus Figur 1 geht hervor, dass die Rohrleitungen sich an den Halbmessern berühren und unmittelbar nebeneinander liegen. Hiermit wird eine optimale Ausnutzung des auf der Fläche, auf die das Rohrreaktorsystem aufgestellt ist, fallenden Lichtes erreicht. Es geht kein Licht, dass bei der üblichen Anordnung von parallel neben einander liegenden und beabstandeten Rohrleitungen zwischen den einzelnen Rohrleitungen hindurch fallen würde, verloren.

Je grösser der Abstand zwischen zwei benachbarten Rohrleitungen, desto kleiner wird die Flächenausnutzung und desto kleiner der Ertrag pro Brutto-Aufstellungsfläche des Photobioreaktors. Je kleiner der Abstand zwischen zwei benachbarten Rohrleitungen, desto grösser wird der Energieaufwand für die Strömung durch die Rohrbögen, weil der Krümmungsradius der Rohrbögen kleiner wird. Die Anordnung von Figur 1 zeigt in einem ersten Ausführungsbeispiel, dass die Rohrbögen 1, 1', 2, 2', 3 und 3' einen Krümmungsradius entsprechend zweieinhalb Rohrleitungsdurchmesser aufweisen. Jeder Rohrbogen des Rohrreaktorsystems stellt die Verbindung her zwischen einem Ende einer ersten Rohrleitung und dem Ende einer weiteren Rohrleitung, die an fünfter Stelle neben der ersten Rohrleitung liegt. Je grösser der Krümmungsradius der Rohrbögen 1, 1', 2, 2', 3 und 3', desto kleiner wird der Strömungswiderstand, der Druckverlust für die Strömung des Mediums in den Rohrleitungen und der Energieaufwand für das Fördern des Mediums durch das Rohrreaktorsystem.

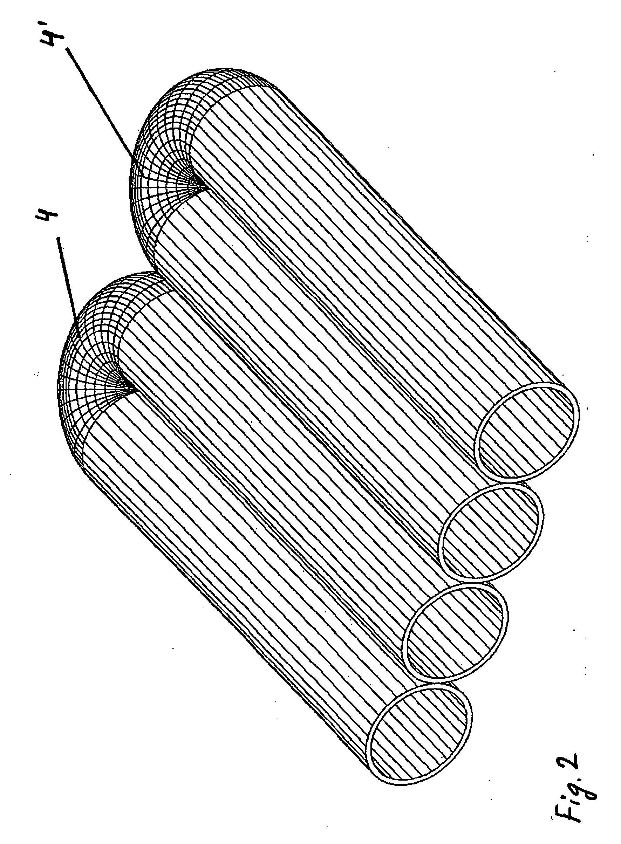

In Figur 2 ist eine Variante des erfindungsgemässen Rohrreaktorsystems dargestellt. Der Krümmungsradius der Rohrbögen 4 und 4' entspricht hier einem halben Rohrleitungsdurchmesser und die Rohrleitungen können unmittelbar angrenzend aneinander aufgestellt werden. Der Druckverlust in den Rohrbögen 4 und 4' gemäss Figur 2 ist etwa viermal so gross als in den Rohrbögen 1, 1', 2, 2', 3 und 3' gemäss Figur 1. Im Vergleich zu einer üblichen Anordnung, wobei der Krümmungsradius einem Rohrleitungsdurchmesser entspricht, und wobei zwischen den Rohrleitungen einen nicht für die Strahlung genutzten Zwischenraum frei bleibt, wird der Druckverlust in der Variante gemäss Figur 1 etwa drei mal so klein.

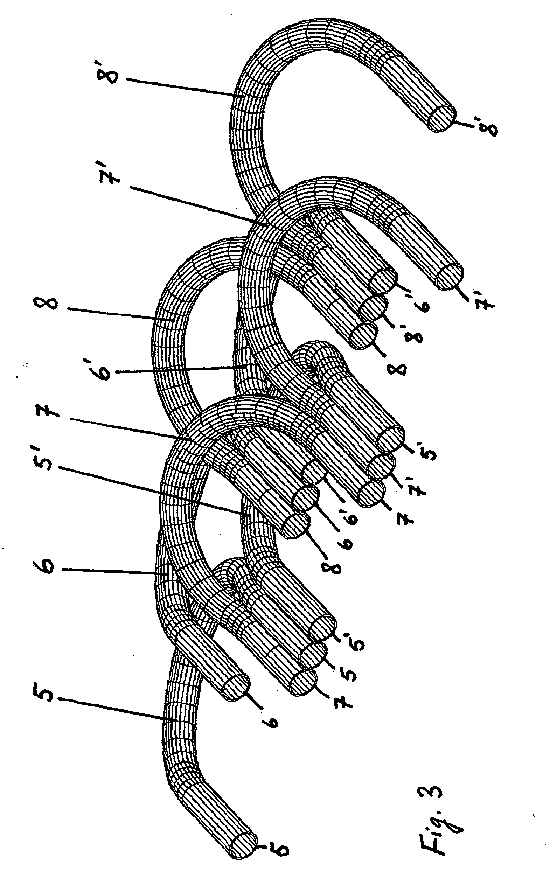

In Figur 3 ist eine weitere Variante des erfindungsgemässen Rohreaktorsystems dargestellt. Hier wurde als Krümmungsradius für die Rohrbögen 5, 5', 6, 6', 7, 7', 8 und 8' dreieinhalb Rohrleitungsdurchmesser gewählt. Der Druckverlust in den Rohrbögen 5, 5', 6, 6', 7, 7', 8 und 8' gemäss Figur 3 ist nochmals etwa 16% kleiner als in den Rohrbögen 1, 1', 2, 2', 3 und 3' gemäss Figur 1. Bei einem Rohrreaktorsystem mit einer grösseren Anzahl von Rohrleitungen wird bei einem Verhältnis von Krümmungsradius zu Rohrleitungsdurchmesser von etwa 7,5 bis 8,5 ein Minimum des Druckverlustes erreicht.

Die Rohrbögen in den Figuren 1 und 3 sind überkreuzend und in mehreren Ebenen angeordnet. Bei den drei Rohrbögen 1, 1', 2, 2', 3 und 3 aus Figur 1 sind drei Ebenen oder drei Anstellwinkel in Bezug auf die Fläche, die durch die Rohrleitungen gebildet wird, ersichtlich. Die Rohrbögen 1 und 1' biegen aus der Ebene der Rohrleitungen nach Unten ab, die Rohrbögen 2 und 2' liegen in der Ebene der Rohrleitungen und die Rohrbögen 3 und 3' biegen aus der Ebene der Rohrleitungen nach Oben ab. Zur Herstellung des Rohrreaktorsystems gemäss Figur 1 wird zusätzlich zum handelsüblichen Rohrbogen 2, 2' ohne Anstellwinkel ein weiterer Rohrbogen 1, 1', 3, 3' mit einem Anstellwinkel von etwa 22,5 Grad nach oben oder nach unten benötigt.

Für die vier Rohrbögen 5, 5', 6, 6', 7, 7', 8 und 8' gemäss Figur 3 werden dementsprechend zwei neue Formen mit Anstellwinkeln von etwa 15 und 30 Grad nach oben oder nach benötigt. Die Rohrbögen 5, 5', 7, 7' sind im Vergleich zu den Rohrbögen 6, 6', 8, 8' mit einem Versatz in der Richtung der Rohrleitungsachsen angeordnet. Bei einem Rohrreaktorsystem gemäss Figur 3, das als ein Mäander mit einer Vielzahl von Rohrschleifen ausgebildet ist, kann dieser Versatz durch einfache Verschiebung von jeweils vier nebeneinander liegenden Rohrleitungen in der Richtung der Rohrleitungsachse erreicht werden. Durch diesen gruppenweise angeordneten Versatz wird erreicht, dass die Anstellwinkel der verschiedenen Rohrbögen möglichst klein gehalten werden können.

Das hier vorgeschlagene Konzept des Rohrreaktorsystems kann beliebig erweitert und variiert werden. Die Rohrbögen werden als thermoplastisches Rohrformteil durch Thermoformen hergestellt. Als Materialien kommen sämtliche thermoplastische Werkstoffe in Frage. Für die Anwendung als Photobioreaktor wird das Rohrreaktorsystem bevorzugt aus durchsichtigen thermoplastischen Materialien, beispielsweise aus PVC-U, hergestellt. Für andere Rohrreaktorsysteme können andere, den jeweiligen Reaktionsbedingungen angepassten Werkstoffe gewählt werden. Die hier dargestellten 180° Rohrbögen können auch jeweils aus zwei 90° Rohrbögen hergestellt werden. Die Rohrbögen werden untereinander und mit den Rohrleitungen durch Kleben, Schweissen oder Stecken verbunden. Der Anstellwinkel der Rohrbögen kann beliebig gewählt werden, wobei auf den niedrigstmöglichen Strömungswiderstand geachtet werden muss.

Statt der dargestellten symmetrischen 180° Rohrbögen mit einem gleichbleibenden Krümmungsradius können die Rohrbögen einen variabel verlaufenden Krümmungsradius aufweisen, wobei der Krümmungsradius vom Bogenanfang zum Bogenende und auch bei dem Uebergang aus der Rohrleitungsebene in den gewinkelt angestellten Rohrbögen strömungsoptimiert gewählt wird. Die Ebene der Rohrleitungen des Photobioreaktors kann, bei diffuser Einstrahlung horizontal oder, wie bei Sonnenkollektoren üblich, senkrecht zum wirksamsten Einfallswinkel des Sonnenlichtes angeordnet werden. Bei ausreichender Intensität der Sonnenstrahlung können auch mehreren Ebenen von Rohrleitungen hinter- oder untereinander angeordnet werden.

## Patentansprüche

1. Rohrreaktorsystem umfassend eine Vielzahl von parallel zueinander angeordneten Rohrleitungen, insbesondere ein Photobioreaktor, wobei die Rohrleitungen an den Rohrenden mittels Rohrbögen zur Bildung von Rohrschleifen miteinander verbindbar angeordnet sind, **dadurch gekennzeichnet, dass** die Rohrleitungen unmittelbar nebeneinander in einer Ebene Tangeordnet sind und dass die Rohrbögen zur Bildung von Rohrschleifen mit voneinander entfernt angeordneten Rohrenden verbindbar ausgebildet sind.

2. Rohrreaktorsystem nach dem Anspruch 1, **dadurch gekennzeichnet, dass** die Rohrbögen einen Krümmungsradius aufweisen, der anderthalb bis achteinhalb Rohrleitungsdurchmesser entspricht, wobei der Krümmungsradius in der Mitte der bogenförmigen Rohrleitung zwischen Rohrbogeninnendurchmesser und Rohrbogenaussendurchmesser gemessen wird.

3. Rohrreaktorsystem nach mindestens einem der Ansprüche 1 oder 2, **dadurch gekennzeichnet, dass** die Rohrbögen in mehreren Ebenen mit unterschiedlichen positiven oder negativen Anstellwinkeln in Bezug auf die Ebene der Rohrleitungen angeordnet sind.

4. Rohrreaktorsystem nach mindestens einem der Ansprüche 1 bis 3, **dadurch gekennzeichnet, dass** die Rohrbögen in Gruppen mit einem Versatz in der Richtung der Rohrleitungsachsen angeordnet sind.

5. Rohrreaktorsystem nach mindestens einem der Ansprüche 1 bis 4, **dadurch gekennzeichnet, dass** die Rohrbögen durch Thermoformen aus thermoplastischem Kunststoffmaterial herstellbar sind.

6. Rohrreaktorsystem nach mindestens einem der Ansprüche 1 bis 5, **dadurch gekennzeichnet, dass** die Rohrbögen aus lichtdurchlässigem PVC-U ausgebildet sind, wobei der PVC-U-Werkstoff für die Prozessbedingungen im Photobioreaktor optimalen Transmissionseigenschaften aufweist.

7. Rohrreaktorsystem nach mindestens einem der Ansprüche 1 bis 6, **dadurch gekennzeichnet, dass** die Rohrbögen jeweils einen 180° Bogen umfassen, wobei der 180° Bogen aus zwei 90° Bogen zusammensetzbar ausgebildet ist.

8. Rohrreaktorsystem nach mindestens einem der Ansprüche 1 bis 7, **dadurch gekennzeichnet, dass** die Rohrbögen durch Kleben, durch Schweissen oder durch Stecken zusammensetzbar ausgebildet sind.

9. Rohrreaktorsystem nach mindestens einem der Ansprüche 1 bis 8, **dadurch gekennzeichnet, dass** die Rohrbögen einen strömungsoptimierten Verlauf des Krümmungsradius aufweisen.

## Claims

1. Tube reactor system comprising a multiplicity of tubular pipes arranged parallel to one another, in particular a photobioreactor, the tubular pipes being arranged so as to be connectable to one another at the tube ends by tube bends for forming tube loops, **characterized in that** the tubular pipes are arranged directly next to one another in a plane, and **in that** the tube bends for forming tube loops are formed so as to be connectable to one another by tube ends arranged at a distance from one another.

2. Tube reactor system according to Claim 1, **characterized in that** the tube bends have a radius of curvature which corresponds to from one and a half to eight and a half times the tubular pipe diameter, the radius of curvature being measured in the middle of the bent tubular pipe between tube bend inner diameter and tube bend outer diameter.

3. Tube reactor system according to at least one of Claims 1 and 2, **characterized in that** the tube bends are arranged in a plurality of planes with different positive and negative setting angles in relation to the plane of the tubular pipes.

4. Tube reactor system according to at least one of Claims 1 to 3, **characterized in that** the tube bends are arranged in groups with an offset in the direction of the tubular pipe axes.

5. Tube reactor system according to at least one of Claims 1 to 4, **characterized in that** the tube bends can be produced by thermo forming from thermoplastic material.

6. Tube reactor system according to at least one of Claims 1 to 5, **characterized in that** the tube bends are formed from translucent PVC-U, the PVC-U material having optimal transmission properties for the process conditions in the photobioreactor.

7. Tube reactor system according to at least one of Claims 1 to 6, **characterized in that** the tube bends respectively comprise a 180° bend, the 180° bend being formed so that it can be composed of two 90° bends.

8. Tube reactor system according to at least one of Claims 1 to 7, **characterized in that** the tube bends are formed so that they can be composed by adhesive bonding, welding or by insertion.

9. Tube reactor system according to at least one of Claims 1 to 8, **characterized in that** the tube bends have a flow-optimized profile of the radius of curvature.

## Revendications

1. Système de réacteur tubulaire comprenant plusieurs conduits tubulaires disposés parallèlement les uns aux autres, en particulier photo-bioréacteur, les conduits tubulaires étant disposés de manière à pouvoir être reliés les uns aux autres aux extrémités des tubes au moyen de courbes tubulaires de manière à former des boucles de tube,
**caractérisé en ce que**
les conduits tubulaires sont disposés directement les uns à côté des autres dans un plan et
**en ce que** les courbes tubulaires destinées à former les boucles de tube sont configurées de manière à pouvoir être reliées à des extrémités de tube séparées les unes des autres.

2. Système de réacteur tubulaire selon la revendication 1, **caractérisé en ce que** les courbes tubulaires présentent un rayon de courbure qui correspond à entre une fois et demi et huit fois et demi le diamètre du conduit tubulaire, le rayon de courbure étant mesuré au milieu du conduit tubulaire courbe, entre le diamètre intérieur de la courbe tubulaire et le diamètre extérieur de la courbe tubulaire.

3. Système de réacteur tubulaire selon au moins l'une des revendications 1 ou 2, **caractérisé en ce que** les courbes tubulaires sont disposées en plusieurs plans à des angles de pose différents, positifs ou négatifs, par rapport au plan des conduits tubulaires.

4. Système de réacteur tubulaire selon au moins l'une des revendications 1 à 3, **caractérisé en ce que** les courbes tubulaires sont disposées en groupes décalés dans la direction de l'axe des conduits tubulaires.

5. Système de réacteur tubulaire selon au moins l'une des revendications 1 à 4, **caractérisé en ce que** les courbes tubulaires peuvent être réalisées par thermoformage d'une matière synthétique thermoplastique.

6. Système de réacteur tubulaire selon au moins l'une des revendications 1 à 5, **caractérisé en ce que** les courbes tubulaires sont formées de PVC-U transparent, le matériau de PVC-U présentant des propriétés de transmission optimales pour les conditions de traitement dans le photo-bioréacteur.

7. Système de réacteur tubulaire selon au moins l'une des revendications 1 à 6, **caractérisé en ce que** les courbes tubulaires comportent chacune une courbe de 180°, la courbe de 180° pouvant être constituée de l'assemblage de deux courbes de 90°.

8. Système de réacteur tubulaire selon au moins l'une des revendications 1 à 7, **caractérisé en ce que** les courbes tubulaires sont configurées de manière à pouvoir être assemblées par collage, par soudage ou par enfichage.

9. Système de réacteur tubulaire selon au moins l'une des revendications 1 à 8, **caractérisé en ce que** les courbes tubulaires présentent une évolution du rayon de courbure optimisée en termes d'écoulement.
